(19) Europäisches Patentamt
European Patent Office
Office européen des brevets

(11) Publication number: 0 013 111
B1

(12) # EUROPEAN PATENT SPECIFICATION

(45) Date of publication of patent specification: 22.02.84

(51) Int. Cl.[3]: C 07 D 498/10, C 07 D 261/08, A 01 N 43/90, C 07 C 135/00 //A01N43/74, (C07D498/10, 307/00, 261/00)

(21) Application number: 79302918.2

(22) Date of filing: 17.12.79

(54) 3′-(substituted phenyl)-spiro(isobenzofuran-1(3H),5′(4′H)-isoxazol)-3-ones, process for their preparation, their use as herbicides and plant growth regulants and their application to the preparation of isoxazole derivatives.

(30) Priority: 20.12.78 US 971462
20.12.78 US 971158

(43) Date of publication of application:
09.07.80 Bulletin 80/14

(45) Publication of the grant of the patent:
22.02.84 Bulletin 84/8

(84) Designated Contracting States:
AT BE CH DE FR GB IT NL SE

(56) References cited:
FR - A - 1 279 643
FR - A - 2 390 441
US - A - 3 258 397

Methoden der organischen Chemie (Houben-Weyl) Band X/3, pages 862-864

(73) Proprietor: MONSANTO COMPANY
Patent Department 800 North Lindbergh Boulevard
St. Louis, Missouri 63166 (US)

(72) Inventor: Howe, Robert Kenneth
12176 Dunsinane Court
Bridgeton, Missouri 63044 (US)
Inventor: Liu, Kou Chang
84-06 98th Street
Woodhaven, New York New York 11421 (US)

(74) Representative: Nash, Brian Walter et al,
Monsanto Europe S.A. Avenue de Tervuren 270-272 Letter Box No 1
B-1150 Brussels (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 013 111 B1

Courier Press, Leamington Spa, England.

3′-(substituted phenyl)-spiro(isobenzofuran-1(3H),5′(4′H)-isoxazol)-3-ones, process for their preparation, their use as herbicides and plant growth regulants and their application to the preparation of isoxazole derivatives

This invention relates to compounds useful as herbicides and plant growth regulants. The compounds may be represented by the formula

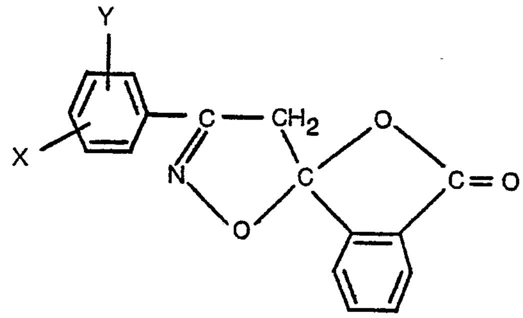

(I)

wherein X and Y are independently selected from hydrogen, halogen, lower alkyl, lower alkoxy, halo-lower-alkyl, phenoxy, phenyl and cyano.

The terms "lower alkyl" and "lower alkoxy" as used herein are understood to mean those alkyl and alkoxy groups having up to five carbon atoms, inclusive. Both straight as well as branched chain alkyl groups are contemplated.

The term "halo-lower-alkyl" as used herein is understood to mean those lower alkyl groups in which at least one, and perhaps all, of the hydrogen atoms have been replaced by halogen atoms. It is to be clearly understood that trifluoromethyl is contemplated as being a halo-lower-alkyl moiety.

The term "halogen" as used herein means chlorine, bromine, fluorine and iodine.

In accordance with one of the novel aspects of the present invention, the compounds of the foregoing formula may be prepared by the reaction of the appropriate nitrile oxide with 3-methylenephthalide. The invention additionally relates to the use of the novel spiro compounds of Formula I as intermediates in the preparation of certain isoxazol-5-yl benzoates which are effective as herbicides and plant growth regulants.

The reaction readily proceeds at room temperature and atmospheric pressure although slightly elevated temperatures and pressures may be utilized to increase the rate of reaction. Although the nitrile oxide may be generated separately and then reacted with 3-methylenephthalide, it has been found to be convenient to generate the nitrile oxide in situ by reacting an appropriate chlorooxime with 3-methylenephthalide under basic conditions as follows:

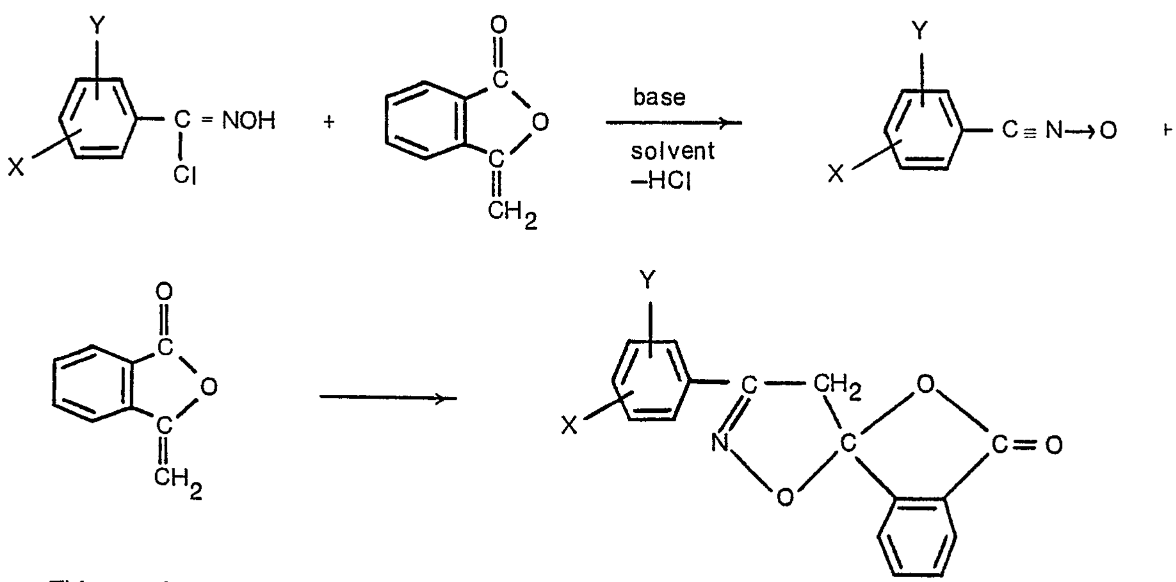


This reaction proceeds at room temperature and atmospheric pressure although slightly elevated temperatures and pressures may be utilized. Normally, an equimolar ratio of reactants is utilized. When the nitrile oxide is relatively unstable, a large excess of chlorooxime may be utilized. Under most conditions, a slight excess is preferred. The molar ratio to be utilized with any given reaction is within the skill of the art. As noted, the reaction proceeds under basic conditions. Bases such as tertiary amines are preferred. Especially preferred is the use of a stoichiometric amount of triethylamine. Any

inert solvent in which the reactants are readily soluble may be utilized. These include ether, dimethylformamide, tetrahydrofuran, chloroform, carbon tetrachloride, methylene chloride and dioxane.

Chlorooximes are known in the art and are readily prepared by reaction of hydroxylamine with the appropriate aryl aldehyde in aqueous alcohol, followed by chlorination of the resultant oxime in accordance with the following scheme:

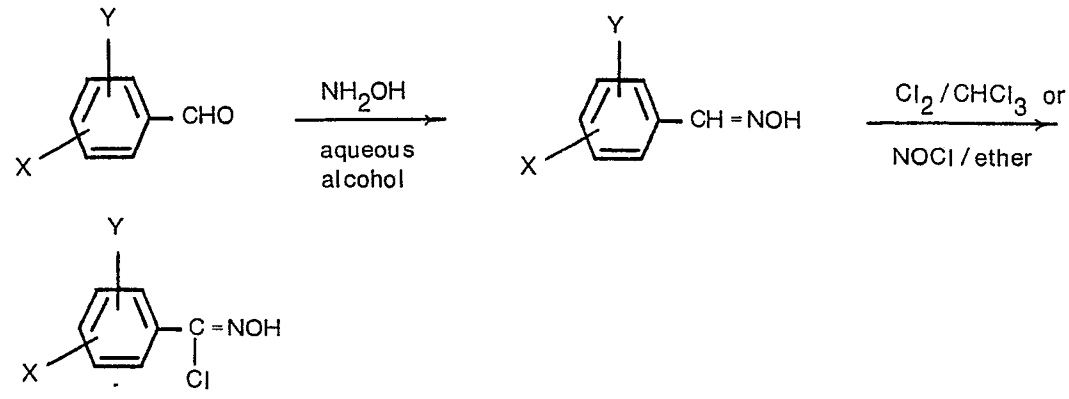


Aryl aldehydes may be prepared by the procedure of Jolad and Rajagopal, *Org, Syn. Coll.*, Vol. V, Page 139 (1973). The procedure of S. V. Vinogradova et al, J. Appl. Chem. (USSR) *44,* 1405 (1971) may be used to prepare 3-methylenephthalide. In order to illustrate the above procedure, the following examples are presented.

### Example 1

Preparation of 3′-(3-Biphenylyl)-Spiro[Isobenzofuran-1(3H),5′(4′H)-Isoxazol]-3-One.

To a solution of 4-biphenylyl-carboxaldehyde chlorooxime (34.8 g, 0.15 mole) and 3-methylenephthalide (14.6 g, 0.1 mole) in 450 ml. of ether was added dropwise triethylamine (15.18 g, 0.15 mole) at 5°C. in 20 minutes. The resultant mixture was stirred at room temperature for 20 hours. The solid was then filtered off. The ethereal solution contained a very small amount of the desired spiro compound according to an NMR analysis. The solid material which was filtered off from the original ether solution was extracted twice with 1200 ml. of chloroform. The chloroform solution was washed twice with water, dried over $CaSO_4$ and concentrated under vacuum to give 24.7 g (72.4%) of a beige solid; m.p. 152—159°C. with decomposition. Recrystallization of 3.5 g of the beige solid from 100 ml. of chloroform gave 1.47 g of white solid; m.p. 170—172°C. with decomposition.

| | | |
|---|---|---|
| Anal. Calc'd. for $C_{22}H_{15}NO_3$: | C, 77.41; | H, 4.43. |
| Found: | C, 77.36; | H, 4.45. |

### Example 2

Preparation of 3′-(4-Chlorophenyl)-Spiro[Isobenzofuran-1(3H),5′(4′H)-Isoxazol]-3-One.

To a solution of 17 g (0.089 mole) of *p*-chlorobenzaldehyde chlorooxime and 14.0 (0.096 mole) 3-methylenephthalide in 300 ml. of ether was added 9.0 g (0.089 mole) of triethylamine in 50 ml. of ether at 0—5°C. during 1 hour. The solution was then allowed to react at room temperature for 20 hours. An infrared spectrum of an aliquot indicated that the reaction was complete. The solid was filtered off. The ethereal solution was washed three times with sodium chloride saturated water, dried over $CaSO_4$ and concentrated under vacuum. Only 2 g of impure product was obtained. The solid material which was filtered off from the original solution was added to 500 ml. of chloroform and washed three times with water. The chloroform solution was cloudy even after 200 ml. of tetrahydrofuran had been added. The chloroform-tetrahydrofuran solution was then dried over $CaSO_4$, filtered and concentrated under vacuum to afford 10.2 g of pale yellow solid (38.2%), m.p. 158—160°C. A colorless solid, m.p. 166—167°C., was obtained after several washes with ether.

| | | | | |
|---|---|---|---|---|
| Anal. Calc'd. for $C_{16}H_{10}NClO_3$: | C, 64.12; | H, 3.36; | N, 4.67; | Cl, 11.83. |
| Found: | C, 64.14; | H, 3.37; | N, 4.67; | Cl, 11.81. |

### Example 3

Preparation of 3′-(2-Methylphenyl)-Spiro[Isobenzofuran-1(3H),5′(4′H)-Isoxazol]-3-One.

To a solution of *o*-tolualdehyde chlorooxime (26.8 g, 0.158 mole), 3-methylenephthalide (14.6 g, 0.1 mole) and ether (400 ml.) was added dropwise a solution of triethylamine (16 g, 0.158 mole) in 30 ml. of ether at 5—10°C. in 45 minutes. The mixture was stirred at room temperature for 40 hours and filtered. The solid material collected was dissolved in 1 liter of chloroform and 200 ml. of water. The chloroform solution was then washed two times with water, dried over $CaSO_4$

and concentrated under vacuum to give 16.4 g of white solid, m.p. 146—149°C. The original ether solution was washed two times with water, dried over $CaSO_4$ and concentrated to 100 ml. Another 1.64 g of the spiro compound precipitated out. The ether solution was then further concentrated to 10.8 g of yellow-brown oil which did not contain a significant amount of the desired product. Recrystallization of 12 g of the crude spiro compound from tetrahydrofuran-ether gave 9.87 g of a pure white solid, m.p. 159—164°C. with decomposition.

| | | |
|---|---|---|
| Anal. Calc'd. for $C_{17}H_{13}NO_3$: | C, 73.11; | H, 4.69; |
| Found: | C, 73.06; | H, 4.74. |

Example 4

Preparation of 3'-(3-Cyanophenyl)-Spiro[Isobenzofuran-1(3H),5'(4'H)-Isoxazol]-3-One.

3'-(3-Cyanophenyl)-spiro[isobenzofuran-1(3H),5'(4'H)-isoxazol]-3-one was prepared according to the procedure of Example 3 in 62.7% isolated yield. The spiro compound obtained was a white solid, m.p. 134—137°C. Recrystallization of 12 g of crude spiro compound from tetrahydrofuran-toluene afforded 6.48 g of white solid, m.p. 165—167°C.

| | | |
|---|---|---|
| Anal. Calc'd. for $C_{17}H_{10}N_2O_3$: | C, 70.34; | H, 3.67. |
| Found: | C, 70.07; | H, 3.50. |

Example 5

Preparation of 3'-(4-Trifluoromethylphenyl)-Spiro-[Isobenzofuran-1(3H),5'(4'H)-Isoxazol]-3-One.

3'-(4-trifluoromethylphenyl)-spiro[isobenzofuran-1(3H),5'(4'H)-isoxazol]-3-one was prepared according to the procedure of Example 3 in 36.8% isolated yield. Recrystallizatoin of 5 g of the crude spiro product from 75 ml. of tetrahydrofuran and 50 ml. of ether gave 3.83 g of pure product as white crystals, m.p. 177—178°C.

| | | |
|---|---|---|
| Anal. Calc'd. for $C_{17}H_{10}F_3NO_3$: | C, 61.27; | H, 3.02. |
| Found: | C, 61.29; | H, 3.06. |

Example 6

Preparation of 3'-(2-Trifluoromethylphenyl)-Spiro-[Isobenzofuran-1(3H),5'(4'H)-Isoxazol]-3-One.

3'(2-trifluoromethylphenyl)-spiro[isobenzofuran-1(3H),5'(4'H)-isoxazol]-3-one was prepared in 35.2% isolated yield according to the procedure of Example 3. The crude spiro product obtained was a white solid, m.p. 149—150.5°C. Recrystallization of 5 g of the solid from tetrahydrofuran (50 ml.) and ether (50 ml.) afforded 4.12 g of colorless needles, m.p. 156—157.5°C.

| | | |
|---|---|---|
| Anal. Calc'd. for $C_{17}H_{10}F_3NO_3$: | C, 61.27; | H, 3.02. |
| Found: | C, 61.24; | H, 3.05. |

Example 7

Preparation of 3'-(3-Methoxyphenyl)-Spiro[Isobenzofuran-1(3H),5'(4'H)-Isoxazol]-3-One.

3'-(3-methoxyhenyl)-spiro[isobenzofuran-1(3H),5'(4'H)-isoxazol]-3-one was prepared following the procedure of Example 3. The original ether solution was washed two times with water, dried over $CaSO_4$ and concentrated to 29.6 g of thick oil. The oil contained 50% of the spiro product according to NMR analysis. Isolation of the spiro compound from the oil was not attempted. The solid material which was filtered off from the original ether solution afforded after workup, 5.56 g (18.8% yield) of the desired product as a yellow solid, m.p. 119.5—123°C. Recrystallization of the solid from tetrahydrofuran-ether gave 4.3 g of a pale yellow solid, m.p. 124—126°C. with decomposition.

| | | |
|---|---|---|
| Anal. Calc'd. for $C_{17}H_{13}NO_4$: | C, 69.15; | H, 4.44. |
| Found: | C, 69.03; | H, 4.50. |

Example 8

Preparation of 3'-(3-Phenoxyphenyl)-Spiro[Isobenzofuran-1(3H),5'(4'H)-Isoxazol]-3-One.

3'-(3-phenoxyphenyl)-spiro[isobenzofuran-1(3H),5'(4'H)-isoxazol]-3-one was prepared in 53.9% isolated yield according to the procedure of Example 3. The crude spiro compound isolated was a yellow oil. Purification of 7.9 g of the oil by high pressure liquid chromatography on silica gel with 50% ethyl acetate — 50% hexane as the eluant gave 4.2 g of the desired product as a white solid, m.p. 114—116°C.

Example 9

Preparation of 3'-(3-Chlorophenyl)-Spiro[Isobenzofuran-1(3H),5'(4'H)-Isoxazol]-3-One.

3'-(3-chlorophenyl-spiro[isobenzofuran-1(3H),5'(4'H)-isoxazol]-3-one was prepared in 86.4% isolated yield according to the procedure of Example 3. The crude product was a gummy material.

Recrystallization of 19 g of the gummy material from tetrahydrofuran-ether gave 5.2 g of solid, m.p. 119—125°C. A sample of 3.5 g of the solid was purified further by high pressure liquid chromatography on a silica gel column with 50% hexane — 50% ethyl acetate as eluant to give 1.77 g of the desired product as a white solid, m.p. 127—129°C.

Anal. Calc'd. for $C_{16}H_{10}ClNO_3$: C, 64.12; H, 3.36.
Found: C, 63.88; H, 3.40.

Example 10

Preparation of 3'-(2,4-Dichlorophenyl)-Spiro[Isobenzofuran-1(3H),5'(4'H)-Isoxazol]-3-One.

By a method similar to the procedure of Example 2, a reaction of 2,4-dichlorobenzaldehyde chlorooxime and 3-methylenephthalide was carried out. The solid collected from the reaction mixture afforded 12.2 g of the desired product after workup, and the ether solution gave 4 g of the desired product (yield 70.2%). Recrystallization from chloroform led to pure white crystals of the desired product, m.p. 158—161°C.

Anal. Calc'd. for $C_{18}H_{13}NCl_2O_3$: C, 57.86; H, 2.73.
Found: C, 57.63; H, 2.74.

Example 11

Preparation of 3'-Phenyl-Spiro[Isobenzofuran-1(3H),5'(4'H)-Isoxazol]-3-One.

3'-Phenyl-spiro[isobenzofuran-1(3H),5'(4'H)-isoxazol]-3-one was prepared according to the procedure of Example 2 in 30% isolated yield, m.p. 103—106°C.

Anal. Calc'd. for $C_{16}H_{11}NO_3$: C, 72.45; H, 4.18.
Found: C, 72.39; H, 4.21.

Example 12

Preparation of 3'-(3-Trifluoromethylphenyl)-Spiro[Isobenzofuran-1(3H),5'(4'H)-Isoxazol]-3-One.

To a solution of 109.5 g (0.75 mole) of 3-methylenephthalide and 285 g (1.27 mole) of *m*-trifluoromethylbenzaldehyde chlorooxime in 3.5 liters of ether was added 128.3 g (1.27 mole) of triethylamine in 250 ml. of ether at 0°C. during 3 hours. The solution was then stirred at room temperature overnight. An infrared spectrum indicated that the reaction was complete. The solid was filtered off. The ethereal solution was washed once with 10% HCl, twice with sodium chloride saturated water, dried over $CaSO_4$ and concentrated under vacuum. Only 19.5 g of impure product was obtained. The solid material which was filtrated from the original reaction solution was stirred with 4 liters of tetrahydrofuran for 4 hours and filtered through a Buchner funnel. The tetrahydrofuran solution obtained was washed once with 10% HCl solution and twice with water saturated with sodium chloride. After being dried and concentrated, the tetrahydrofuran solution gave 140.6 g of pure product, m.p. 170°C.

Anal. Calc'd. for $C_{17}H_{10}F_3NO_3$: C, 61.27; H, 3.02.
Found: C, 61.25; H, 3.04.

Example 13

Preparation of 3'-(*p*-Fluorophenyl)-Spiro[Isobenzofuran-1(3H),5'(4'H)-Isoxazol]-3-One.

3'-(*p*-fluorophenyl)-spiro[isobenzofuran-1(3H),5'(4'H)-isoxazol]-3-one was prepared according to the procedure of Example 1 in 65% isolated yield, m.p. 142—145°C.

Anal. Calc'd. for $C_{16}H_{10}FNO_3$: C, 67.84; H, 3.56.
Found: C, 67.89; H, 3.59.

In acordance with one aspect of the present invention, the spiro compounds of the foregoing formula have been found to be effective as herbicides. The compounds may used by themselves or as the active ingredient in a herbicidal composition.

As used herein, the term "herbicidal active ingredient" is understood to mean a spiro compound of the foregoing formula (I).

Control of undesirable weed growth may be obtained by applying the herbicidal active ingredient to the plant locus which is defined herein to include the growth medium surrounding the plant, the seeds, emerging seedlings, roots, stems, leaves, flowers and other plant parts.

To illustrate the herbicidal properties of the compounds of the present invention, said compounds were tested in the following manner.

The pre-emergent test was conducted as follows:

A good grade of top soil was placed in aluminum pans and compacted to a depth of .95 to 1.27 cm. from the top of the pan. On the top of the soil was placed a predetermined number of seeds or

vegetative propagules of various plant species which are compacted to a soil level. The soil required to level fill the pans after seeding or adding vegetative propagules was weighed into a pan. A known amount of the herbicidal active ingredient applied in a solvent or as a wettable powder and the soil were thouroughly mixed, and used as a cover layer for prepared pans. After treatment, the pans were moved into a greenhouse bench where they were watered from below as needed to give adequate moisture for germination and growth.

As noted in Tables I and II, below, approximately 2 or 4 weeks after seeding and treating, the plants were observed to determine all deviations from the normal growth habit and the results recorded. A herbicidal rating code was used to signify the extent of phytotoxicity of each species. The ratings are defined as follows:

| % Control | Rating |
|---|---|
| 0—24 | 0 |
| 25—49 | 1 |
| 50—74 | 2 |
| 75—100 | 3 |

The plant species utilized in these tests are identified by letter in accordance with the following legend:

| | |
|---|---|
| A — Canada Thistle | K — Barnyardgrass |
| B — Cocklebur | L — Soybean |
| C — Velvetleaf | M — Sugar Beet |
| D — Morningglory | N — Wheat |
| E — Lambsquarters | O — Rice |
| F — Smartweed | P — Sorghum |
| G — Yellow Nutsedge | Q — Wild Buckwheat |
| H — Quackgrass | R — Hemp Sesbania |
| I — Johnsongrass | S — Panicum Spp |
| J — Downy Brome | T — Crabgrass |

Results of the pre-emergent tests are summarized in Tables I and II, below.

TABLE I

| Compound of Example No. | WAT* | kg/h | Pre-Emergent Plant Species A | B | C | D | E | F | G | H | I | J | K |
|---|---|---|---|---|---|---|---|---|---|---|---|---|---|
| 1 | 2 | 11.2 | 0 | 0 | 0 | 2 | 1 | 2 | 0 | 0 | 0 | 1 | 3 |
| 2 | 4 | 11.2 | 3 | 2 | 3 | 3 | 3 | 3 | 2 | 3 | 0 | 1 | 3 |
| 3 | 4 | 11.2 | 3 | 2 | 3 | 3 | 3 | 2 | 3 | 3 | 0 | 2 | 2 |
| 4 | 4 | 11.2 | 1 | 1 | 3 | 3 | 2 | 2 | 1 | 2 | 0 | 2 | 1 |
| 5 | 2 | 11.2 | 0 | 0 | 1 | 1 | 2 | 3 | 0 | 2 | 0 | 1 | 2 |
| 6 | 4 | 11.2 | 3 | 2 | 2 | 2 | 2 | 2 | 2 | 2 | 0 | 2 | 3 |
| 7 | 4 | 11.2 | 3 | 1 | 2 | 3 | 3 | 3 | 2 | 2 | 0 | 2 | 3 |
| 8 | 4 | 11.2 | 3 | — | 1 | 1 | 3 | 3 | 0 | 0 | 0 | 0 | 3 |
| 9 | 4 | 11.2 | 3 | 1 | 2 | 3 | 3 | 1 | 2 | 3 | 1 | 3 | 3 |
| 10 | 4 | 11.2 | 0 | 0 | 1 | — | 2 | 1 | 0 | 1 | 0 | 2 | 2 |
| 11 | 4 | 11.2 | 3 | 2 | 3 | 3 | 3 | 2 | 1 | 2 | 1 | 1 | 3 |
| 12 | 4 | 11.2 | 3 | 2 | 3 | 3 | 3 | 3 | 2 | 3 | 0 | 2 | 3 |
| 13 | 4 | 11.2 | 2 | — | 2 | 2 | 2 | 2 | 0 | 3 | 2 | 1 | 3 |

* — Weeks after treatment.

TABLE II

| Compound of Example No. | WAT* | kg/h | L | M | N | O | P | B | Q | D | R | E | F | C | J | S | K | T |
|---|---|---|---|---|---|---|---|---|---|---|---|---|---|---|---|---|---|---|
| | | | Pre-Emergent Plant Species | | | | | | | | | | | | | | | |
| 1 | 2 | 5.6 | 0 | 1 | 0 | 1 | 1 | 0 | 0 | 1 | 3 | 3 | 3 | 0 | 2 | 0 | 3 | 3 |
| 1 | 2 | 1.12 | 0 | 0 | 0 | 0 | 0 | 0 | 0 | 1 | 2 | 3 | 1 | 0 | 0 | 0 | 2 | 2 |
| 2 | 4 | 5.6 | 1 | 2 | 1 | 2 | 2 | 1 | 1 | 2 | 1 | — | 3 | 2 | 1 | 0 | 3 | 0 |
| 2 | 2 | 1.12 | 0 | 2 | 0 | 1 | 0 | 0 | 0 | 2 | 0 | — | 0 | 0 | 1 | 0 | 0 | 0 |
| 2 | 2 | 0.28 | 0 | 2 | 0 | 0 | 0 | 0 | 0 | 0 | 0 | — | 0 | 0 | 0 | 0 | 0 | 0 |
| 3 | 4 | 5.6 | 0 | 3 | 3 | 2 | 2 | 1 | 0 | 0 | 1 | 3 | 2 | 3 | 3 | 3 | 3 | 3 |
| 3 | 4 | 1.12 | 0 | 2 | 2 | 1 | 1 | 1 | 1 | 2 | 0 | 3 | 0 | 2 | 2 | 2 | 2 | 2 |
| 3 | 2 | 0.28 | 1 | 2 | 1 | 1 | 0 | 0 | 1 | 0 | 0 | 3 | 1 | 0 | 0 | 0 | 1 | 2 |
| 4 | 4 | 5.6 | 1 | 3 | 1 | 2 | 2 | 0 | 3 | 1 | 3 | 3 | 3 | 2 | 0 | 1 | 1 | 3 |
| 4 | 2 | 1.12 | 1 | 3 | 0 | 0 | 0 | 0 | 2 | 0 | 1 | 3 | 1 | 1 | 0 | 0 | 1 | 1 |
| 4 | 2 | 0.28 | 0 | 1 | 0 | 0 | 0 | 0 | 0 | 0 | 0 | 2 | 0 | 0 | 0 | 0 | 0 | 1 |
| 5 | 4 | 5.6 | 2 | 3 | 1 | 1 | 2 | 0 | 3 | 1 | 2 | 3 | 3 | 1 | 1 | 0 | 3 | 1 |
| 5 | 2 | 1.12 | 0 | 1 | 0 | 1 | 0 | 0 | 2 | 0 | 0 | 3 | 3 | 0 | 0 | 0 | 0 | 3 |
| 6 | 4 | 5.6 | 1 | 2 | 0 | 1 | 0 | 1 | 2 | 0 | 1 | 2 | 3 | 1 | 0 | 1 | 2 | 3 |
| 6 | 4 | 1.12 | 0 | 2 | 0 | 2 | 1 | 0 | 1 | 1 | 2 | 2 | 3 | 0 | 3 | 0 | 2 | 3 |
| 6 | 2 | 0.28 | 0 | 1 | 0 | 1 | 0 | 0 | 1 | 1 | 0 | 0 | 1 | 0 | 0 | 0 | 1 | 1 |
| 7 | 4 | 5.6 | 3 | 3 | 2 | 3 | 2 | 2 | 2 | 2 | 2 | 2 | 3 | 2 | 2 | 2 | 3 | 3 |
| 7 | 4 | 1.12 | 0 | 2 | 0 | 0 | 0 | 0 | 0 | 2 | 0 | 0 | 0 | 1 | 3 | 0 | 0 | 0 |

TABLE II (Cont'd)

| Compound of Example No. | WAT* | kg/h | L | M | N | O | P | B | Q | D | R | E | F | C | J | S | K | T |
|---|---|---|---|---|---|---|---|---|---|---|---|---|---|---|---|---|---|---|
| | | | Pre-Emergent Plant species | | | | | | | | | | | | | | | |
| 7 | 2 | 0.28 | 0 | 1 | 0 | 0 | 0 | 0 | 0 | 0 | 1 | 0 | 2 | 0 | 1 | 0 | 0 | 2 |
| 7 | 2 | 0.056 | 0 | 1 | 0 | 0 | 0 | 0 | 0 | 0 | 0 | 1 | 1 | 0 | 0 | 0 | 0 | 1 |
| 8 | 2 | 5.6 | 0 | 1 | 0 | 0 | 0 | 3 | 2 | 3 | 2 | 3 | 0 | 0 | 0 | 0 | 3 | 2 |
| 8 | 2 | 1.12 | 0 | 1 | 0 | 0 | 0 | — | 1 | 3 | 0 | 1 | 1 | 1 | 0 | 0 | 3 | 2 |
| 8 | 2 | 0.28 | 3 | 1 | 0 | 0 | 0 | 3 | 0 | 0 | 0 | 1 | 1 | 0 | 0 | 0 | 2 | 2 |
| 9 | 4 | 5.6 | 3 | 3 | 1 | 3 | 2 | 1 | 2 | 2 | 3 | 3 | 2 | 3 | 2 | 3 | 3 | 3 |
| 9 | 4 | 1.12 | 1 | 3 | 0 | 1 | 0 | 0 | 3 | 3 | 2 | 3 | 3 | 1 | 0 | 0 | 0 | 1 |
| 9 | 4 | 0.28 | 0 | 2 | 0 | 2 | 0 | 0 | 0 | 3 | 1 | 2 | 3 | 2 | 2 | 2 | 2 | 3 |
| 10 | 2 | 5.6 | 1 | 3 | 1 | 2 | 1 | — | 0 | 0 | 1 | 3 | 3 | 2 | 1 | 1 | 2 | 3 |
| 10 | 2 | 1.12 | 1 | 2 | 0 | 2 | 0 | — | 0 | 0 | 1 | 3 | 1 | 0 | 1 | 1 | 3 | 1 |
| 10 | 2 | 0.28 | 0 | 2 | 0 | 2 | 0 | — | 0 | — | 0 | 2 | 1 | 0 | 1 | 0 | 1 | 2 |
| 11 | 4 | 5.6 | 2 | 3 | 0 | 2 | 1 | 0 | 0 | 3 | 3 | 3 | 2 | 2 | 0 | 1 | 3 | 1 |
| 11 | 4 | 1.12 | 0 | 3 | 0 | 1 | 0 | 0 | 0 | 2 | 3 | 3 | 3 | 2 | 0 | 0 | 1 | 0 |
| 11 | 2 | 0.28 | 0 | 0 | 0 | 1 | 0 | 0 | 1 | 1 | 3 | 1 | 1 | 1 | 0 | 0 | 0 | 1 |
| 12 | 4 | 5.6 | 3 | 3 | 1 | 3 | 2 | 1 | 3 | 3 | 3 | 3 | 3 | 2 | 1 | 2 | 3 | 3 |
| 12 | 4 | 1.12 | 2 | 3 | 0 | 3 | 1 | 1 | 3 | 2 | 3 | 3 | 2 | 2 | 0 | 0 | 2 | 3 |
| 12 | 2 | 0,28 | 3 | 1 | 0 | 1 | 1 | 1 | 1 | 2 | 3 | 3 | 1 | 2 | 0 | 0 | 1 | 3 |
| 13 | 4 | 5.6 | 2 | 3 | 1 | 2 | 3 | — | 3 | 1 | 3 | 3 | 3 | 1 | 1 | 0 | 3 | 3 |
| 13 | 4 | 1.12 | 1 | 2 | 1 | 1 | 2 | — | 0 | — | 1 | 0 | 0 | 1 | 0 | 1 | 1 | 1 |
| 13 | 2 | 0.28 | 1 | 1 | 0 | 0 | 0 | — | 0 | 0 | 0 | 1 | 1 | 0 | 0 | 0 | 0 | 0 |

* — Weeks after treatment.

The post-emergent tests were conducted as follows:

The herbicidal active ingredients are applied in spray form to two or three-week old specimens of various plant species. The spray, a solution or wettable powder suspension containing the appropriate rate of herbicidal active ingredient to give the desired test rate and a surfactant, is applied to the plants. The treated plants are placed in a greenhouse and approximately two or four weeks later the effects are observed and recorded. The results are shown in Tables III and IV in which the post-emergent herbicidal rating code is as follows:

| % Control | Rating |
|---|---|
| 0—24 | 0 |
| 25—49 | 1 |
| 50—74 | 2 |
| 75—99 | 3 |
| 100 | 4 |

The plant species utilized in these tests are identified by letter in accordance with the previous legend.

TABLE III

| Compound of Example No. | WAT* | kg/h | Post-Emergent Plant Species A | B | C | D | E | F | G | H | I | J | K |
|---|---|---|---|---|---|---|---|---|---|---|---|---|---|
| 1 | 2 | 11.2 | 0 | 1 | 0 | 1 | 1 | 0 | 0 | 0 | 0 | 0 | 0 |
| 2 | 4 | 11.2 | 1 | 2 | 1 | 1 | — | 0 | 0 | 0 | 0 | 0 | 0 |
| 3 | 4 | 11.2 | 2 | 1 | 1 | 1 | 1 | 2 | 1 | 0 | 1 | 1 | 2 |
| 4 | 4 | 11.2 | 1 | 1 | 1 | 1 | 1 | 0 | 0 | 0 | 0 | 0 | 0 |
| 5 | 2 | 11.2 | 1 | 0 | 1 | 1 | 1 | 1 | 0 | 0 | 0 | 0 | 0 |
| 6 | 4 | 11.2 | 1 | 2 | 1 | 2 | 1 | 1 | 0 | 0 | 0 | 0 | 0 |
| 7 | 4 | 11.2 | 1 | 2 | 2 | 2 | 1 | 3 | 1 | 0 | 0 | 0 | 2 |
| 8 | 4 | 11.2 | 1 | 2 | 1 | 2 | 2 | 2 | 0 | 0 | 0 | 0 | 0 |
| 9 | 4 | 11.2 | 4 | 2 | 2 | 1 | 2 | 4 | 1 | 0 | 2 | 1 | 1 |
| 10 | 4 | 11.2 | 1 | 1 | 1 | 1 | 1 | 0 | 1 | 0 | 0 | 0 | 0 |
| 11 | 4 | 11.2 | 2 | 2 | 2 | 2 | 2 | 3 | 1 | 1 | 1 | 1 | 1 |
| 12 | 4 | 11.2 | 2 | 2 | 2 | 3 | 2 | 4 | 2 | 2 | 1 | 0 | 2 |
| 13 | 4 | 11.2 | 2 | 2 | 2 | 2 | 2 | 1 | 0 | 0 | 2 | 0 | 1 |

* — Weeks after treatment.

TABLE IV

| Compound of Example No. | WAT* | kg/h | Pre-Emergent Plant Species L | M | N | O | P | B | Q | D | R | E | F | C | J | S | K | T |
|---|---|---|---|---|---|---|---|---|---|---|---|---|---|---|---|---|---|---|
| 7 | 4 | 5.6 | 2 | — | 1 | 0 | 0 | 2 | 2 | 2 | 2 | 1 | 4 | 2 | 1 | 0 | 1 | 0 |
| 7 | 2 | 1.12 | 2 | 1 | 0 | 0 | 0 | 2 | 2 | 2 | 2 | 1 | 3 | 2 | 0 | 1 | 1 | 1 |
| 7 | 2 | 0.28 | 1 | — | 0 | 0 | 0 | 1 | 1 | 1 | 1 | 1 | 2 | 1 | 0 | 0 | 1 | 0 |
| 9 | 4 | 5.6 | 2 | 2 | 1 | 1 | 2 | 2 | 4 | 2 | 4 | 3 | 4 | 2 | 1 | 1 | 2 | 1 |
| 9 | 4 | 1.12 | 2 | 1 | 0 | 1 | 0 | 2 | 2 | 2 | 2 | 2 | 4 | 2 | 0 | 0 | 1 | 0 |
| 9 | 2 | 0.056 | 0 | 1 | 0 | 1 | 0 | 0 | 0 | 0 | 0 | 3 | 2 | 0 | 1 | 0 | 0 | 1 |
| 11 | 4 | 5.6 | 2 | 2 | 1 | 0 | 2 | 2 | 2 | 2 | — | 4 | 4 | 2 | 1 | 0 | 1 | 2 |
| 11 | 4 | 1.12 | 2 | 1 | 0 | 0 | 0 | 2 | — | 2 | 2 | 4 | 2 | 2 | 0 | 0 | 1 | 0 |
| 12 | 4 | 5.6 | 2 | 2 | 0 | 1 | 1 | 2 | 3 | 2 | — | 2 | — | 2 | 0 | 1 | 2 | 0 |
| 12 | 4 | 1.12 | 2 | 1 | 0 | 0 | 1 | — | 2 | 2 | — | 2 | 2 | 2 | 0 | 1 | 2 | 1 |
| 12 | 2 | 0.056 | 0 | 2 | 0 | 0 | 0 | 0 | 2 | 0 | 1 | 1 | 1 | 1 | 0 | 0 | 1 | 2 |
| 12 | 2 | 0.01 | 0 | 0 | 0 | 0 | 0 | — | 0 | 0 | 0 | 1 | 0 | 0 | 1 | 1 | 0 | 2 |

* — Weeks after treatment.

The above tables illustrate one aspect of the present invention, that is, the use of the compounds of the invention to kill or injure undesirable plants, e.g., weeds. Another aspect of the invention, however, is the use of the spiro compounds of formula (I) for the regulation of desirable plant growth, especially leguminous plants such as soybeans. More particularly, it has been found that compounds of the foregoing formula (I) have been found to be effective in regulating the growth of leguminous plants.

As used herein, the regulation of "plant growth or development" is understood to mean the modification of the normal sequential development of a treated desirable plant to agricultural maturity. Such modifications are most readily observed as changes in size, shape, color or texture of the treated plant or any of its parts. Similarly, changes in the quantity of plant fruit or flowers are also quite apparent from visual inspection. The above changes may be characterized as an acceleration or retardation of plant growth, stature reduction, leaf or canopy alteration, increased branching, terminal inhibition, increased flowering, defoliation, increased root growth, increased cold hardiness and the like. While many of these modifications are desirable in and of themselves, most often it is their effect on the economic result that is of most importance. For example, a reduction in stature of the plant permits the growing of more plants per unit area. A darkening of the foliar color may be illustrative of higher chlorophyll activity indicative of improved rate of photosynthesis.

Although the regulation of plant growth in accordance with the present invention may include partial inhibition of plant growth when used as a plant growth regulant, it does not include the total inhibition or killing of such plants. The present invention contemplates the use of an amount of the spiro compounds of formula (I) as the active ingredient in a plant growth regulating composition which will modify the normal sequential development of the treated plant to agricultural maturity. Such plant growth regulating amounts may vary, not only with the material selected, but also with the modifying effect desired, the species of plant and its stage of development, the plant growth medium and whether a permanent or a transitory effect is sought. It is, however, well within the skill of the art to determine the amount of active ingredient required.

Modification of the plants may be accomplished by applying the active ingredient to the plant locus which has been defined herein to include the growth medium surrounding the plant, the seeds, emerging seedlings, roots, stems, leaves, flowers, or other plant parts. Such application may be made directly to the plant part, or indirectly by application of the plant growth medium.

Utilizing the spiro compounds of formula (I) as the active ingredient in a plant growth regulating composition, said compounds were found to possess plant growth regulating activity when tested in accordance with the following procedure.

A number of soybean plants, variety Williams, were grown from seeds in plastic pots in the greenhouse for a period of one week at which time the plants are thinned to one plant per pot. When the second trifoliate leaf (three weeks) was fully expanded, the plants were treated with a solution of the active ingredient in acetone and water. Aqueous Tween 20 was used as a surfactant (Tween is a registered Trade Mark).

When the fifth trifoliate leaf (four to five weeks) was fully expanded, the treated plants were compared with the non-treated control plants and the observations recorded. Those observations are summarized in Table V below.

TABLE V

| Compound of Example N°. | Rate (kg/h) | Observations |
|---|---|---|
| 1 | 2.8 | Stature reduction, leaf alteration of old new growth, leaf inhibition, axillary bud inhibition, inhibition of dry weight. |
| 1 | 0.56 | Stature reduction, leaf alteration of old and new growth, leaf inhibition, axillary bud inhibition, inhibition of dry weight. |
| 1 | 0.112 | Stature reduction, leaf alteration of old and new growth, leaf inhibition, axillary bud inhibition, inhibition of dry weight. |
| 2 | 2.8 | Stature reduction, dark foliar color, leaf inhibition, leaf distortion of old and new growth, inhibition of dry weight. |
| 2 | 0.56 | Stature reduction, dark foliar color, leaf inhibition, leaf distortion of old and new growth, inhibition of dry weight. |

TABLE V (Cont'd)

| Compound of Example N°. | Rate (kg/h) | Observations |
| --- | --- | --- |
| 2 | 0.112 | Stature reduction, altered canopy, leaf inhibition, leaf alteration of old and new growth, inhibition of dry weight. |
| 3 | 2.8 | Stature reduction, altered canopy, leaf inhibition, leaf alteration of old and new growth, inhibition of dry weight. |
| 3 | 0.56 | Leaf alteration of old and new growth, inhibition of dry weight. |
| 3 | 0.112 | Inhibition of dry weight. |
| 4 | 2.8 | Stature reduction, inhibition of apical development, leaf inhibition, leaf alteration of new growth, epinasty, inhibition of dry weight. |
| 4 | 0.56 | Stature reduction, inhibition of apical development, leaf inhibition, leaf alteration of new growth, epinasty, inhibition of dry weight. |
| 4 | 0.112 | Stature reduction, inhibition of apical development, leaf inhibition, leaf alteration of new growth, epinasty, inhibition of dry weight. |
| 5 | 2.8 | Stature reduction, leaf inhibition, leaf alteration of old and new growth, epinasty, inhibition of dry weight. |
| 5 | 0.56 | Stature reduction, thick leaf texture, leaf alteration of old and new growth, epinasty, inhibition of dry weight. |
| 5 | 0.112 | Stature reduction, altered canopy, leaf alteration of old and new growth, epinasty, inhibition of dry weight. |
| 6 | 2.8 | Stature reduction, leaf alteration of old and new growth, dark foliar color, leaf inhibition, inhibition of dry weight. |
| 6 | 0.56 | Stature reduction, leaf alteration of old and new growth, dark foliar color, leaf inhibition, inhibition of dry weight. |
| 6 | 0.112 | Stature reduction, leaf alteration of old and new growth, leaf inhibition, inhibition of dry weight. |
| 7 | 2.8 | Stature reduction, inhibition of apical development, dark foliar color, thick leaf texture, leaf inhibition, inhibition of dry weight. |

TABLE V (Cont'd)

| Compound of Example N°. | Rate (kg/h) | Observations |
|---|---|---|
| 7 | 0.56 | Stature reduction, inhibition of apical development, dark foliar color, leaf inhibition, leaf alteration of new growth, inhibition of dry weight. |
| 7 | 0.112 | Stature reduction, inhibition of apical development, leaf inhibition, leaf alteration of old and new growth, inhibition of dry weight. |
| 8 | 2.8 | Leaf alteration of old and new growth, leaf inhibition, inhibition of dry weight. |
| 8 | 0.56 | Leaf alteration of old and new growth. |
| 8 | 0.112 | None |
| 9 | 2.8 | Stature reduction, inhibition of apical development, dark foliar color, thick leaf texture, leaf inhibition, inhibition of dry weight. |
| 9 | 0.56 | Stature reduction, inhibition of apical development, dark foliar color, thick leaf texture, leaf inhibition, inhibition of dry weight. |
| 9 | 0.112 | Stature reduction, inhibition of apical development, dark foliar color, leaf inhibition, leaf alteration of new growth, inhibition of dry weight. |
| 10 | 2.8 | Leaf alteration, leaf inhibition. |
| 10 | 0.56 | Leaf alteration. |
| 10 | 0.112 | None. |
| 11 | 2.8 | Stature reduction, inhibition of apical development, dark foliar color, thick leaf texture, leaf inhibition, inhibition of dry weight. |
| 11 | 0.56 | Stature reduction, dark foliar color, thick leaf texture, altered canopy, leaf inhibition, inhibition of dry weight. |
| 11 | 0.112 | Stature reduction, dark foliar color, thick leaf texture, altered canopy, leaf inhibition, inhibition of dry weight. |
| 12 | 2.8 | Stature reduction, leaf distortion of new growth, dark foliar color, thick leaf texture, leaf inhibition, inhibition of dry weight. |
| 12 | 0.56 | Stature reduction, leaf distortion of new growth, dark foliar color, thick leaf texture, leaf inhibition, inhibition of dry weight. |

TABLE V (Cont'd)

| Compound of Example N°. | Rate (kg/h) | Observations |
|---|---|---|
| 12 | 0.112 | Stature reduction, leaf distortion of new growth, dark foliar color, thick leaf texture, leaf inhibition, inhibition of dry weight. |
| 13 | 2.8 | Stature reduction, leaf alteration, leaf inhibition, leaf distortion of new growth, inhibition of dry weight. |
| 13 | 0.56 | Stature reduction, leaf alteration, leaf inhibition, leaf distortion of new growth, inhibition of dry weight. |
| 13 | 0.112 | Leaf alteration of old and new growth, leaf inhibition. |

As can be seen from the above data, the spiro compounds of the invention are especially effective in reducing the stature of soybean plants as well as altering the leaf morphology.

Thus, the above data illustrate that the compounds of the invention may be used as a herbicide or a plant growth regulant. When used as a herbicide, it is desirable that rates of application about 1.12 kilograms per hectare and above be utilized. When used to regulate the growth of desirable plants, rates below 5.6 kilograms per hectare, especially .056 to 2.8, are preferred.

In selecting the appropriate time and rate of application of the active ingredient, it will be recognized that precise rates will also be dependent upon the desired response, mode of application, plant variety, soil conditions and various other factors known to those skilled in the art. In addition, it will be recognized that single or multiple applications may be used to exert the desired response.

In the practice of the invention, the active ingredient, whether used as a herbicide or a plant growth regulant, can be used alone or in combination with other pesticides or a material referred to in the art as an adjuvant in either liquid or solid form. To prepare such compositions, the active ingredient is admixed with an adjuvant including diluents, extenders, carriers and conditioning agents to provide compositions in the form of finely-divided particulate solids, granules, pellets, wettable powders, dusts, solutions and aqueous dispersions or emulsions. Thus, the active ingredient can be used with an adjuvant such as a finely-divided particulate solid, a solvent liquid of organic origin, water, a wetting agent, dispersing agent or emulsifying agent or any suitable combination of these.

Illustrative finely-divided solid carriers and extenders which are useful in the compositions of this invention include the talcs, clays, pumice, silica, diatomaceous earth, quartz, fullers earth, sulfur, powdered cork, powdered wood, walnut flour, chalk, tobacco dust and charcoal. Typical liquid diluents include Stoddard solvent, acetone, alcohols, glycols, ethyl acetate and benzene. The plant growth regulating compositions of this invention, particularly liquids and wettable powders, usually contain one or more surface-active agents in amounts sufficient to render a given composition readily dispersible in water or in oil. The term "surface-active agent" is understood to include wetting agents, dispersing agents, suspending agents and emulsifying agents. Such surface-active agents are well known and reference is made to U.S. Patent N°. 2,547,724, columns 3 and 4, for detailed examples of the same.

Generally, the active ingredients are applied in the form of a composition containing one or more adjuvants which aid in the application of a uniform distribution of the active ingredient. The application of liquid and particulate solid compositions of the active ingredient can be carried out by conventional techniques utilizing, for example, spreaders, powder dusters, boom and hand sprayers and spray dusters. The compositions can also be applied from airplanes as a dust or spray.

Compositions of this invention, whether used as a herbicide or a plant growth regulant, generally contain from about 5 to 95 parts active ingredient, about 1 to 50 parts surface-active agent and about 4 to 94 parts solvents, all parts being by weight based on the total weight of the composition.

This invention also relates to the preparation of isoxazol-5-yl benzoates that are useful in agriculture. Belgian Patent 837,454 discloses such compounds to be effective plant growth regulants. The isoxazol-5-yl benzoates are prepared by conversion of isoxazolin-5-yl benzoate with N-bromosuccinimide or dichlorodicyanobenzoquinone. Isoxazolin-5-yl benzoates are prepared, however, from vinyl benzoates which are somewhat difficult to prepare.

In accordance with the present invention, 3-Aryl-isoxazol-5-yl-benzoic acid and esters thereof having the formula

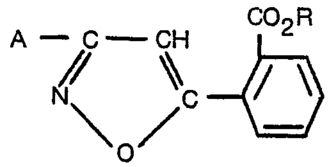

wherein R is hydrogen or $R'CH_2$— and R' is hydrogen or alkyl having up to four carbon atoms inclusive, A is pentafluorophenyl or a group having the formula

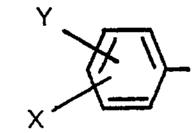

wherein X and Y are independently selected from hydrogen, halogen, $C_{1-5}$ alkyl, $C_{1-5}$ alkoxy, halo-$C_{1-5}$ alkyl, phenoxy and phenyl, are prepared by reacting in the presence of a catalytic amount of a strong acid, a spiro compound having the formula

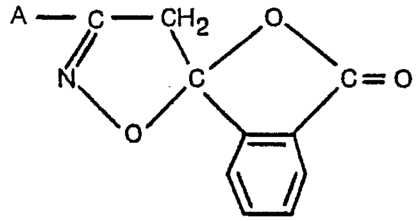

wherein A, X and Y are as defined above, with ROH at a temperature ranging from room temperature to the reflux temperature of the ROH compound. When R' is alkyl having up to four carbon atoms, said alkyl group may have a straight or a branched chain.

The specific acid used is not critical. However the use of stronger acids increases the rate of reaction. Accordingly, strong acids such as hydrochloric acid, sulfuric acid, methanesulfonic acid and toluenesulfonic acid are preferred.

Although the reaction may be conducted at room temperature, it is preferred to conduct it at or near the reflux temperature of the hydroxy compound.

The reaction may be conducted above atmospheric pressure. However, atmospheric pressure is preferred.

It should be noted that the invention contemplates the preparation of 3-Aryl-isoxazol-5-yl benzoic acid in addition to esters thereof. The free acid may be prepared in one of two ways. First, the spiro compound may be cleaved utilizing aqueous acid without any alcohol being present. In other words, the above reaction is utilized in which R is hydrogen. The free acid is also prepared by reaction of the spiro compound with an alcohol at low temperatures. At low temperatures and/or short residence times, the preparation of free acid is favored. Thus, a mixture containing both the free acid and the ester may be formed. The amount of ester prepared in accordance with the novel process is dependent upon the temperature of the reaction and the length of time that the reaction is allowed to proceed. To prepare the ester, it is preferable to conduct the reaction at or near the reflux temperature of the hydroxy compound for a sufficient period of time in which to convert the spiro compound to the ester. Under such conditions, only minor amounts of the free acid are produced. If lower temperatures are utilized, a greater amount of the free acid would be produced. When preparing esters, it is preferable to use an excess amount of the hydroxy compound.

Generally, it has been found that the spiro compound is soluble in the hydroxy compound. If the spiro compound is not readily soluble in said hydroxy compound, it may be beneficial to utilize an inert solvent.

In order to illustrate the novel aspects of the present invention, the following examples are presented.

Example 14

Preparation of Methyl 2-(3-Pentafluorophenyl-5-Isoxazolyl)Benzoate.

A solution of 3'-(pentafluorophenyl)spiro[isobenzofuran-1(3H),5'(4'H)-isoxazol]-3-one (3.9 g., 0.0106 mole), concentrated $H_2SO_4$ (0.3 ml.) and methanol (100 ml.) was held at reflux for 30 hours, cooled and poured into 600 ml. of ice water. The mixture was extracted with 1 liter of ether. The organic extract was then washed two times with water, dried over $CaSO_4$ and concentrated under vacuum to 3.8 g. of crystals; m.p. 96.5—98°C. Recrystallization of the crude ester from toluene-hexane gave 2.96 g. of pure product as colorless crystals; m.p. 97—98°C.

| | | |
|---|---|---|
| Anal. Calc'd. for $C_{17}H_{18}F_5NO_3$: | C, 55.30; | H, 2.18. |
| Found: | C, 55.27; | H, 2.21. |

## Example 15

Preparation of Methyl 2-[3-(*p*-Trifluoromethylphenyl)-5-Isoxazolyl]Benzoate.

Methyl 2-[3-(*p*-trifluoromethylphenyl)-5-isoxazolyl]benzoate was prepared according to the procedure of Example 14 in 82% yield as a colorless oil. the oil was crystallized from toluene-hexane to give 2.7 g. of white crystals; m.p. 90.5—92°C.

| | | |
|---|---|---|
| Anal. Calc'd. for $C_{18}H_{12}F_3NO_3$: | C, 62.25; | H, 3.48. |
| Found: | C, 62.28; | H, 3.51. |

## Example 16

Preparation of Methyl 2-[3-(*o*-Methylphenyl)-5-Isoxazolyl]Benzoate.

Methyl 2-[3-(*o*-methylphenyl)-5-isoxazolyl]benzoate was prepared according to the procedure of Example 14 as a colorless oil in 95% yield. The oil (3.99 g.) was purified by column chromatography on silica gel with 50% toluene — 50% ethyl acetate as the eluant. One fraction contained 2.49 g. of pure product, $n_D^{25.8} = 1.6017$.

| | | |
|---|---|---|
| Anal. Calc'd. for $C_{18}H_{15}NO_3$: | C, 73.71; | H, 5.15. |
| Found: | C, 73.52; | H, 5.20. |

## Example 17

Preparation of Methyl 2-[3-[3-(Trifluoromethyl)Phenyl]-5-Isoxazolyl]Benzoate.

A mixture of 2 g. (0.006 mole) of 3'-(*m*-trifluoromethylphenyl)-spiro[isobenzofuran-1(3H),5'(4'H)-isoxazol]-3-one, 2 ml. of concentrated surfuric acid and 50 ml. of methanol was heated at reflux for 17 hours. The solution was then poured into 100 g. of ice and extracted twice with 300 ml. of ether. The ether solutions were combined, washed twice with water saturated sodium chloride, and dried over $CaSO_4$. Removal of the solvent gave 2 g. of colorless viscous liquid, which was crystallized from hexane at 0°C. to give colorless crystals; m.p. 46—48°C.

## Example 18

Preparation of Methyl 2-[3-(*p*-Chlorophenyl)-5-Isoxazolyl]Benzoate.

A solution of 6.3 g. (0.020 mole) of 3'-(*p*-chlorophenyl)-spiro[isobenzofuran-1(3H),5'(4'H)-isoxazol]-3-one, 2 ml. of concentrated $H_sSO_4$ and 300 ml. of methanol was stirred at reflux for 20 hours. The cooled solution was poured into ice water (3 liters) and extracted with ether. The combined organic extracts were washed twice with water, dried over $CaSO_4$ and concentrated under vacuum to yield 5.8 g. of colorless crystals (88%) of product. Recrystallization from hexane afforded 5.4 g. of an analytically pure sample; m.p. 89.5—91°C.

| | | |
|---|---|---|
| Anal. Calc'd. for $C_{17}H_{12}NClO_3$: | C, 65.14; | H, 3.86. |
| Found: | C, 65.14; | H, 3.90. |

## Example 19

Preparation of Methyl 2-[3-(2,4-Dichlorophenyl)-5-Isoxazolyl]Benzoate.

Methyl 2-[3-(2,4-dichlorophenyl)-5-isoxazolyl]benzoate was prepared in 94% yield with the same procedure as Example 18. Recrystallization of 4.7 g. of the crude product once from hexane and once from heptane yielded 3.3 g. pure crystals of the benzoate; m.p. 94—96°C.

| | | |
|---|---|---|
| Anal. Calc'd. for $C_{17}H_{11}NCl_2O_3$: | C, 58.64; | H, 3.18. |
| Found: | C, 58.66; | H, 3.19. |

## Example 20

Preparation of Ethyl 2-[3-(2,4-Dichlorophenyl)-5-Isoxazolyl]Benzoate.

A solution of 4 g. (0.011 mole) of 3'-(2,4-dichlorophenyl)-spiro[isobenzofuran-1(3H),5'(4'H)-isoxazol]-3-one, 2 ml. of concentrated $H_2SO_4$ and 200 ml. of ethanol was held at reflux for 19 hours, cooled, and poured into ice water. The resultant mixture was extracted twice with ether. The ethereal solution was washed twice with water, dried over $CaSO_4$ and concentrated under vacuum to give an oil. The oil was dissolved in hot hexane to afford 4.2 g. of a crystalline solid (96.9%). Recrystallization of the solid from hexane gave 3.1 g. of pure product; m.p. 85—86°C.

| | | |
|---|---|---|
| Anal. Calc'd. for $C_{18}H_{13}NCl_2O_3$: | C, 59.69; | H, 3.62. |
| Found: | C, 59.66; | H, 3.64. |

## Example 21

Preparation of Methyl 2-[3-(3-Phenoxyphenyl)-5-Isoxazolyl]Benzoate.

Methyl 2-[3-(3-phenoxyphenyl)-5-isoxazolyl]benzoate was prepared in accordance with Example 5 in 95% yield as a light brown oil. Chromatography on silica gel with 50% cyclohexane — 5% ethyl

acetate as the eluant afforded pure product as a colorless oil; ir ($CHCl_3$) 1722 $cm^{-1}$; $n_D^{26.8} = 1.6217$.

| | | |
|---|---|---|
| Anal. Calc'd. for $C_{23}H_{17}NO_4$: | C, 74.38; | H, 4.61. |
| Found: | C, 74.20; | H, 4.68. |

Example 22

Preparation of n-Butyl 2-[3-(3-Trifluoromethylphenyl)-5-Isoxazolyl]Benzoate.

*n*-Butyl 2-[3-(3-trifluoromethylphenyl)-5-isoxazolyl]benzoate was prepared according to the procedure of Example 18 (utilizing *n*-butanol in lieu of methanol) in 94.8% yield as a pale yellow oil. The oil (5.54 g.) was dissolved in 25 ml. of hexane and cooled in dry ice to obtain a white solid at low temperature. This solid melted at room temperature and was chromatographed on a silica gel column with ethyl acetate as the eluant. 3.8 g. of pure product was obtained as a colorless oil; ir ($CHCl_3$) 1720 $cm^{-1}$; $n_D^{26.6} = 1.5412$.

| | | |
|---|---|---|
| Anal. Calc'd. for $C_{21}H_{18}F_3NO_3$: | C, 64.78; | H, 4.66. |
| Found: | C, 64.78; | H, 4.66. |

Example 23

Preparation of n-Pentyl 2-[3-(3-Trifluoromethylphenyl)-5-Isoxazolyl]Benzoate.

A solution of 3'-(*m*-trifluoromethylphenyl)-spiro[isobenzofuran-1(3H),5'(4'H)-isoxazol]-3-one (10 g., 0.03 mole) and concentrated $H_2SO_4$ (1 ml.) in *n*-pentyl alcohol (150 ml.) was held at reflux for 14 hours, cooled and poured into 300 ml. of ice water. The mixture was extracted with 600 ml. of ether. The ethereal solution was washed two times with water, dried over $CaSO_4$ and concentrated under vacuum to give 12.05 g. of product as a yellow oil (99%). The oil was chromatographed on silica gel using ethyl acetate as the eluant. 8.4 g. of colorless oil was collected; ir ($CHCl_3$) 1725 $cm^{-1}$; $n_D^{25} = 1.537$.

| | | |
|---|---|---|
| Anal. Calc'd. for $C_{22}H_{20}NF_3O_3$: | C, 65.50; | H, 5.00. |
| Found: | C, 65.53; | H, 5.06. |

Example 24

Preparation of Ethyl 2-[3-(trifluoromethylphenyl)-5-Isoxazolyl]Benzoate.

Ethyl 2-[3-(trifluoromethylphenyl)-5-isoxazolyl]benzoate was obtained in 97.7% yield as a viscous oil by the method described in Example 23 (ethanol used in lieu of *n*-pentyl alcohol). Crystallization of 5.3 g. of the oil from heptane-toluene yielded 3.7 g. of colorless crystals; m.p. 28—30°C.

| | | |
|---|---|---|
| Anal. Calc'd. for $C_{19}H_{14}F_3NO_3$: | C, 63.16; | H, 3.91. |
| Found: | C, 63.13; | H, 3.93. |

Example 25

Preparation of 2-[3-(3-Trifluoromethylphenyl)-5-Isoxazolyl]Benzoic Acid.

A mixture of 0.28 g. of 3'(3-trifluoromethylphenyl)-spiro[isobenzofuran-1(3H),5'(4'H)-isoxazol]-3-one, 0.5 ml. of concentrated HCl, 10 ml. of water and 6 ml. of dioxane was stirred at reflux for one hour. The mixture was cooled and filtered to give 0.25 g. of white solid; m.p. 176.5—178°C. in 89% yield.

Example 26

Preparation of 2-[3-(2-Methylphenyl)-5-Isoxazolyl]Benzoic Acid.

A mixture of 0.26 g. of 3'-(2-methylphenyl)-spiro[isobenzofuran-1(3H),5'(4'H)-isoxazol]-3-one, 4 ml. of water, 4 ml. of dioxane and 0.10 ml. of concentrated sulfuric acid was stirred at reflux for one hour, allowed to cool and diluted with 15 ml. of water. The resultant oil was stirred several minutes to give 0.24 g. of white solid; m.p. 162—163.5°C. in 92% yield.

The above examples disclose, therefore, an efficient process for preparing isoxazol-5-yl benzoic acid and esters thereof that are useful as herbicides and plant growth regulants.

**Claims**

1. A compound having the formula

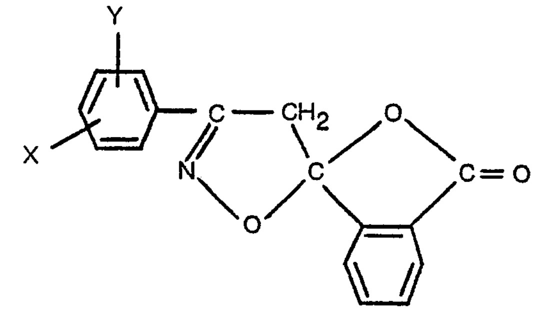

wherein X and Y are independently selected from hydrogen, halogen, $C_{1-5}$ alkyl, $C_{1-5}$ alkoxy, halo-$C_{1-5}$-alkyl, phenoxy, phenyl and cyano.

2. A compound according to Claim 1 wherein X is hydrogen and Y is halogen, $C_{1-5}$ alkyl, $C_{1-5}$ alkoxy, halo-$C_{1-5}$-alkyl, phenoxy, phenyl and cyano.

3. A compound according to Claim 1 wherein said halo-$C_{1-5}$-alkyl is trifluoromethyl.

4. A compound according to Claim 1 wherein X is hydrogen and Y is chloro in the *para* position.

5. A compound according to Claim 1 wherein X is hydrogen and Y is fluoro in the *para* position.

6. A compound according to Claim 1 wherein X is hydrogen and Y is phenoxy in the *meta* position.

7. A compound according to Claim 3 wherein X is hydrogen and Y is trifluoromethyl in the *meta* position.

8. A method of regulating the growth of desirable leguminous plants which comprises applying to the plant locus a plant growth regulating effective amount of a compound having the formula

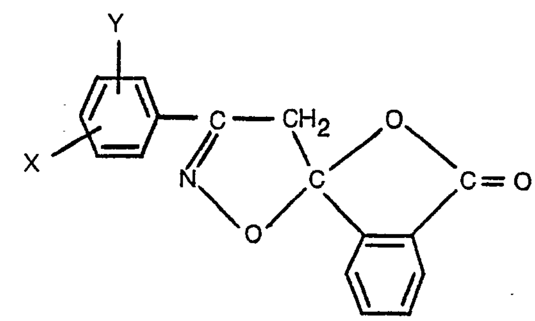

wherein X and Y are independently selected from the group consisting of hydrogen, halogen, $C_{1-5}$ alkyl, $C_{1-5}$ alkoxy, halo-$C_{1-5}$-alkyl, phenoxy, phenyl and cyano.

9. A method according to Claim 8 wherein X is hydrogen and Y is halogen, $C_{1-5}$ alkyl, $C_{1-5}$ alkoxy, halo-$C_{1-5}$-alkyl, phenoxy, phenyl and cyano.

10. A method according to Claim 8 wherein said halo-$C_{1-5}$-alkyl is trifluoromethyl.

11. A method according to Claim 8 wherein X is hydrogen and Y is chloro in the *para* position.

12. A method according to Claim 8 wherein X is hydrogen and Y is fluoro in the *para* position.

13. A method according to Claim 8 wherein X is hydrogen and Y is phenoxy in the *meta* position.

14. A method according to Claim 10 wherein X is hydrogen and Y is trifluoromethyl in the *meta* position.

15. A method for preventing the growth of undesirable plants which comprises applying to the plant locus a herbicidally effective amount of a compound as defined in Claim 8.

16. A method according to Claim 15 wherein X is hydrogen and Y is halogen, $C_{1-5}$ alkyl, $C_{1-5}$ alkoxy, halo-$C_{1-5}$-alkyl, phenoxy, phenyl and cyano.

17. A method according to Claim 15 wherein said halo-$C_{1-5}$-alkyl is trifluoromethyl.

18. A method according to Claim 15 wherein X is hydrogen and Y is chloro in the *para* position.

19. A method according to Claim 15 wherein X is hydrogen and Y is fluoro in the *para* position.

20. A method according to Claim 15 wherein X is hydrogen and Y is phenoxy in the *meta* position.

21. A method according to Claim 17 wherein X is hydrogen and Y is trifluoromethyl in the *meta* position.

22. An agricultural chemical composition which comprises from about 5 to about 95 parts by weight of a compound as defined in claim 1; the remaining parts being comprised of one or more suitable adjuvants, carriers and/or diluents.

23. A composition according to Claim 22 wherein X is hydrogen and Y is halogen, $C_{1-5}$ alkyl, $C_{1-5}$ alkoxy, halo-$C_{1-5}$-alkyl, phenoxy, phenyl and cyano.

24. A composition according to Claim 22 wherein said halo-$C_{1-5}$-alkyl is trifluoromethyl.

25. A composition according to Claim 22 wherein X is hydrogen and Y is chloro in the *para* position.

26. A composition according to Claim 22 wherein X is hydrogen and Y is fluoro in the *para* position.

27. A composition according to Claim 22 wherein X is hydrogen and Y is phenoxy in the *meta* position.

28. A composition according to Claim 24 wherein X is hydrogen and Y is trifluoromethyl in the *meta* position.

29. A process for preparing compounds as defined in claim 8 which comprises reacting a nitrile oxide having the formula

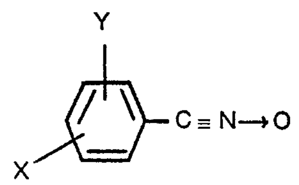

with 3-methylenephthalide in an inert solvent.

30. A process according to Claim 29 wherein said nitrile oxide is prepared in situ by adding a chlorooxime having the formula

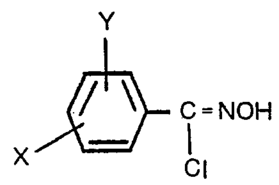

and a base to the 3-methylenephthalide solution.

31. A process according to Claim 29 wherein said base is a tertiary amine.

32. A process according to Claim 31 wherein said amine is triethylamine.

33. A process for preparing 3-Aryl-isoxazol-5-yl-benzoic acid and esters thereof having the formula

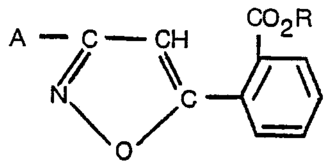

wherein R is hydrogen or R'$CH_2$— and R' is hydrogen or alkyl having up to four carbon atoms inclusive, A is pentafluorophenyl or a group having the formula

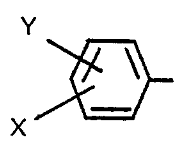

wherein X and Y are independently selected from the group consisting of hydrogen, halogen, $C_{1-5}$ alkyl, $C_{1-5}$ alkoxy, halo $C_{1-5}$ alkyl, phenoxy and phenyl, which comprises reacting in the presence of a catalytic amount of a strong acid, a spiro compound having the formula

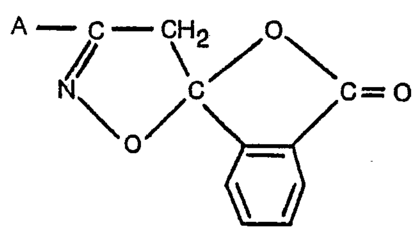

wherein A, X and Y are as defined above, with ROH at a temperature ranging from room temperature to the reflux temperature of the ROH compound.

34. A process according to Claim 33 wherein said temperature is the reflux temperature of the ROH compound.

35. A process according to Claim 33 wherein R is $R'CH_2$—.

36. A process according to Claim 33 wherein Y is trifluoromethyl.

37. A process according to Claim 33 wherein the strong acid is sulfuric acid.

**Revendications**

1. Composé ayant la formule:

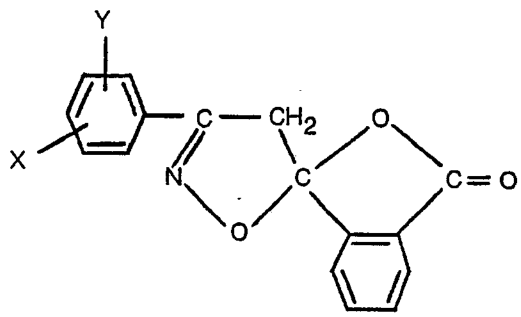

où X et Y sont indépendamment choisis parmi l'hydrogène, un halogène, un groupe alkyle en $C_{1-5}$, alcoxy en $C_{1-5}$, haloalkyle en $C_{1-5}$, phénoxy, phényle et cyano.

2. Composé selon la revendication 1, dans lequel X est l'hydrogène et Y est un halogène, un groupe alkyle en $C_{1-5}$, alcoxy en $C_{1-5}$, haloalkyle en $C_{1-5}$, phénoxy, phényle et cyano.

3. Composé selon la revendication 1, dans lequel le groupe haloalkyle en $C_{1-5}$ est le groupe trifluorométhyle.

4. Composé selon la revendication 1, dans lequel X est l'hydrogène et Y est le chlore en position para.

5. Composé selon la revendication 1, dans lequel X est l'hydrogène et Y est le fluor en position para.

6. Composé selon la revendication 1, dans lequel X est l'hydrogène et Y est le groupe phénoxy en position méta.

7. Composé selon la revendication 3, dans lequel X est l'hydrogène et Y est le groupe trifluorométhyle en position méta.

8. Procédé pour la régulation de la croissance de plantes légumineuses souhaitables, qui consiste à appliquer au lieu des plantes une quantité, efficace pour la régulation de la croissance des plantes, d'un composé ayant la formule:

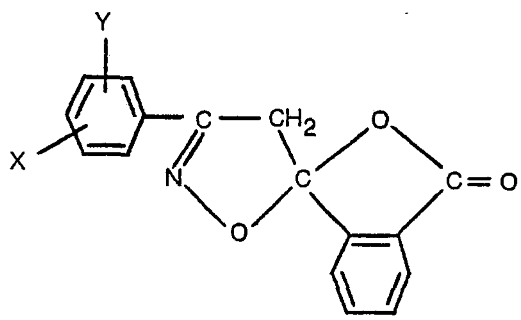

où X et Y sont indépendamment choisis dans le groupe se composant d'hydrogène, de groupe alkyle en $C_{1-5}$, alcoxy en $C_{1-5}$, haloalkyle en $C_{1-5}$, phénoxy, phényle et cyano.

9. Procédé selon la revendication 8, dans lequel X est l'hydrogène et Y est un halogène, un groupe alkyle en $C_{1-5}$, alcoxy en $C_{1-5}$, haloalkyle en $C_{1-5}$, phénoxy, phényle et cyano.

10. Procédé selon la revendication 8, dans lequel le groupe haloalkyle en $C_{1-5}$ est le groupe trifluorométhyle.

11. Procédé selon la revendication 8, dans lequel X est l'hydrogène et Y est le chlore en position para.

12. Procédé selon la revendication 8, dans lequel X est l'hydrogène et Y est le fluor en position para.

13. Procédé selon la revendication 8, dans lequel X est l'hydrogène et Y est le groupe phénoxy en position méta.

14. Procédé selon la revendication 10, dans lequel X est l'hydrogène et Y est le groupe trifluorométhyle en position méta.

15. Procédé pour empêcher la croissance de plantes indésirables, qui consiste à appliquer au lieu des plantes une quantité, efficace du point de vue herbicide, d'un composé tel que défini dans la revendication 8.

16. Procédé selon la revendication 15, dans lequel X est l'hydrogène et Y est un halogène, un groupe alkyle en $C_{1-5}$, alcoxy en $C_{1-5}$, haloalkyle en $C_{1-5}$, phénoxy, phényle et cyano.

17. Procédé selon la revendication 15, dans lequel le groupe haloalkyle en $C_{1-5}$ est le groupe trifluorométhyle.

18. Procédé selon la revendication 15, dans lequel X est l'hydrogène et Y est le chlore en position para.

19. Procédé selon la revendication 15, dans lequel X est l'hydrogène et Y est le fluor en position para.

20. Procédé selon la revendication 15, dans lequel X est l'hydrogène et Y est le groupe phénoxy en position méta.

21. Procédé selon la revendication 17, dans lequel X est l'hydrogène et Y est le groupe trifluorométhyle en position méta.

22. Composition chimique pour l'agriculture, qui comprend environ 5 à environ 95 parties en poids d'un composé comme défini dans la revendication 1, les parties restantes étant composés d'un ou de plusieurs adjuvants, supports et/ou diluants.

23. Composition selon la revendication 22, dans laquelle X est l'hydrogène et Y est un halogène, un groupe alkyle en $C_{1-5}$, alcoxy en $C_{1-5}$, haloalkyle en $C_{1-5}$, phénoxy, phényle et cyano.

24. Composition selon la revendication 22, dans laquelle le groupe haloalkyle en $C_{1-5}$ est le groupe trifluorométhyle.

25. Composition selon la revendication 22, dans laquelle X est l'hydrogène, et Y est le chlore en position para.

26. Composition selon la revendication 22, dans laquelle X est l'hydrogène et Y est le fluor en position para.

27. Composition selon la revendication 22, dans laquelle X est l'hydrogène et Y est le groupe phénoxy en position méta.

28. Composition selon la revendication 24, dans laquelle X est l'hydrogène et Y est le groupe trifluorométhyle en position méta.

29. Procédé de préparation de composés comme définis dans la revendication 8, qui consiste à faire réagir un oxyde de nitrile ayant la formule:

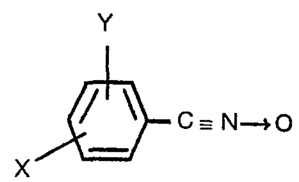

avec le 3-méthylènephtalide dans un solvant inerte.

30. Procédé selon la revendication 29, dans lequel l'oxyde de nitrile est préparé in situ en ajoutant une chlorooxime ayant la formule:

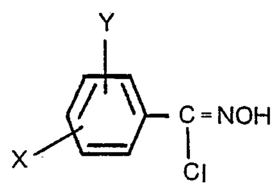

et une base à la solution de 3-méthylènephtalide.

31. Procédé selon la revendication 29, dans lequel la base est une amine tertiaire.

32. Procédé selon la revendication 31, dans lequel l'amine est la triéthylamine.

33. Procédé de préparation d'acide 3-aryl-isoxazol-5-yl-benzoïque et de ses esters ayant la formule:

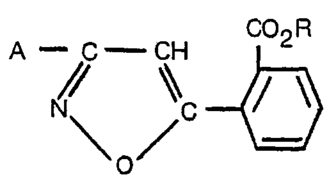

où est l'hydrogène ou $R'CH_2$— et R' est l'hydrogène ou un groupe alkyle ayant jusqu'à 4 atomes de carbone inclusivement, A est le groupe pentafluorophényle ou un groupe ayant la formule:

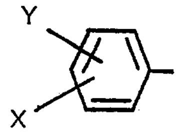

où X est Y sont indépendamment choisis dans le groupe se composant d'hydrogène, d'halogène, de groupe alkyle en $C_{1-5}$, alcoxy en $C_{1-5}$, haloalkyle en $C_{1-5}$, phénoxy et phényle, qui consiste à faire réagir en présence d'une quantité catalytique d'un acide fort, un composé spiro ayant la formule:

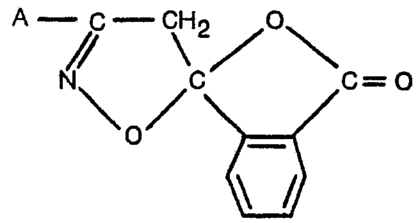

où A, X et Y sont comme définis ci-dessus, avec ROH à une température allant de la température ambiante à la température de reflux du composé ROH.

34. Procédé selon la revendication 33, dans lequel la température est la température de reflux du composé ROH.

35. Procédé selon la revendication 33, dans lequel R est $R'CH_2$—.

36. Procédé selon la revendication 33, dans lequel Y est le groupe trifluorométhyle.

37. Procédé selon la revendication 33, dans lequel l'acide fort est l'acide sulfurique.

**Patentansprüche**

1. Verbindung der Formel

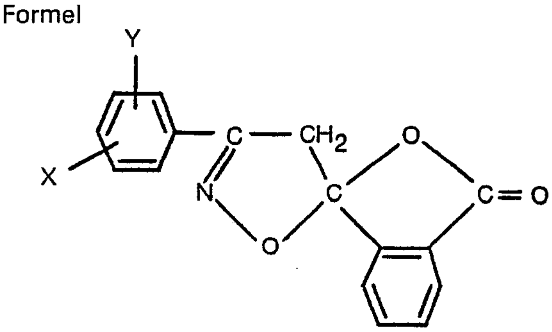

worin X und Y unabhängig voneinander ein Wasserstoff- oder Halogenatom oder einen $C_{1-5}$-Alkyl-, $C_{1-5}$-Alkoxy-, Halogen-$C_{1-5}$-alkyl-, Phenoxy-, Phenyl oder Cyanorest bedeuten.

2. Verbindung nach Anspruch 1, worin X ein Wasserstoffatom darstellt und Y ein Halogenatom, einen $C_{1-5}$-Alkyl-, $C_{1-5}$Alkoxy-, halogen-$C_{1-5}$-alkyl-, Phenoxy-, Phenyl- oder Cyanorest bedeutet.

3. Verbindung nach Anspruch 1, worin der Halogen-$C_{1-5}$-alkylrest die Trifluoromethylgruppe ist.

4. Verbindung nach Anspruch 1, worin X ein Wasserstoffatom darstellt und Y ein Chloratom in der para-Stellung bedeutet.

5. Verbindung nach Anspruch 1, worin X ein Wasserstoffatom bedeutet und Y ein Fluoratom in der para-Stellung darstellt.

6. Verbindung nach Anspruch 1, worin X ein Wasserstoffatom bedeutet und Y einen Phenoxyrest in der meta-Stellung darstellt.

7. Verbindung nach Anspruch 3, worin X ein Wasserstoffatom darstellt und Y eine Trifluormethylgruppe in der meta-Position bedeutet.

8. Verfahren zur Regulierung des Wachstums erwüschter Hülsenfrucht-Pflanzen, welches auf den Pflanzenort die Aufbringung einer das Pflanzenwachstum regulierenden effektiven Menge einer Verbindung der Formel

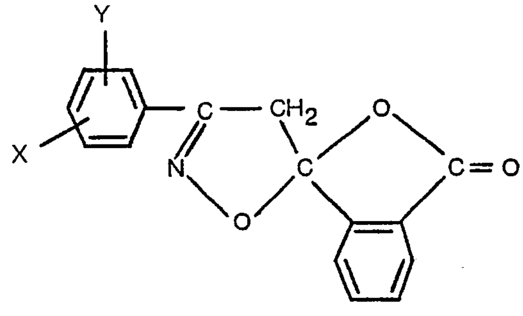

vorsieht, worin X und Y unabhängig voneinander ein Wasserstoff- oder Halogenatom oder einen $C_{1-5}$-Alkyl-, $C_{1-5}$-Alkoxy-, Halogen-$C_{1-5}$-alkyl-, Phenoxy-, Phennyl- oder Cyanorest bedeuten.

9. Verfahren nach Anspruch 8, worin X ein Wasserstoffatom darstellt und Y ein Halogenatom, einen $C_{1-5}$-Alkyl-, $C_{1-5}$-Alkoxy-, Halogen-$C_{1-5}$alkyl-, Phenoxy-, Phenyl- oder Cyanorest darstellt.

10. Verfahren nach Anspruch 8, worin der Halogen-$C_{1-5}$-alkylrest die Trifluormethylgruppe bedeutet.

11. Verfahren nach Anspruch 8, worin X ein Wasserstoffatom darstellt und Y ein Chloratom in der para-Stellung bedeutet.

12. Verfahren nach Anspruch 8, worin X ein Wasserstoffatom darstellt und Y ein Fluoratom in der para-Stellung bedeutet.

13. Verfahren nach Anspruch 8, worin X ein Wasserstoffatom darstellt und Y einen Phenoxyrest in der meta-Stellung bedeutet.

14. Verfahren nach Anspruch 10, worin X ein Wasserstoffatom bedeutet und Y einen Trifluormethylrest in der meta-Stellung darstellt.

15. Verfahren zur Verhinderung des Wachstums unerwünschter Pflanzen, welches die Aufbringung einer herbicid wirksamen Menge einer Verbindung gemäß der Definition in Anspruch 8 auf den Pflanzenort umfaßt.

16. Verfahren nach Anspruch 15, worin X ein Wasserstoffatom bedeutet und Y ein Halogenatom, eine $C_{1-5}$-Alkyl-, $C_{1-5}$-Alkoxy-, Halogen-$C_{1-5}$-alkyl-, Phenoxy-, Phenyl- oder Cyanogruppe bedeutet.

17. Verfahren nach Anspruch 15, worin der Halogen-$C_{1-5}$-alkylrest die Trifluormethylgruppe darstellt.

18. Verfahren nach Anspruch 15, worin X ein Wasserstoffatom bedeutet und Y ein Chloratom in der para-Stellung darstellt.

19. Verfahren nach Anspruch 15, worin X ein Wasserstoffatom bedeutet und Y ein Fluoratom in der para-Stellung darstellt.

20. Verfahren nach Anspruch 15, worin X ein Wasserstoffatom darstellt und Y eine Phenoxygruppe in der meta-Stellung bedeutet.

21. Verfahren nach Anspruch 17, worin X ein Wasserstoffatom bedeutet und Y eine Trifluormethylgruppe in der meta-Stellung darstellt.

22. Chemische Zusammensetzung für die Landwirtschaft, welche von etwa 5 bis etwa 95 Gewichtsteile einer Verbindung gemäß der Definition in Anspruch 1 enthält, wobei die verbleibenden Teile von einem oder mehreren geeigneton Zusatzstoffen, Trägern und/oder Verdünnungsmitteln gebildet werden.

23. Zusammensetzung nach Anspruch 22, worin X ein Wasserstoffatom bedeutet und Y ein Halogenatom, einen $C_{1-5}$-Alkyl-, $C_{1-5}$-Alkoxy-, Halogen-$C_{1-5}$-alkyl-, Phenoxy-, Phenyl- oder Cyanorest bedeutet.

24. Zusammensetzung nach Anspruch 22, worin der Halogen-$C_{1-5}$-alkylrest die Trifluormethylgruppe ist.

25. Zusammensetzung nach Anspruch 22, worin X ein Wasserstoffatom darstellt und Y ein chloratom in der para-Stellung bedeutet.

26. Zusammensetzung nach Anspruch 22, worin X ein Wasserstoffatom darstellt und Y ein Fluoratom in der para-Stellung bedeutet.

27. Zusammensetzung nach Anspruch 22, worin X ein Wasserstoffatom bedeutet und Y einen Phenoxyrest in der meta-Stellung darstellt.

28. Zusammensetzung nach Anspruch 24, worin X ein Wasserstoffatom darstellt und Y ein Trifluormethylgruppe in der meta-Stellung bedeutet.

29. Verfahren zur Herstellung von Verbindungen gemäß der Definition in Anspruch 8, welches die Umsetzung eines Nitriloxids der Formel

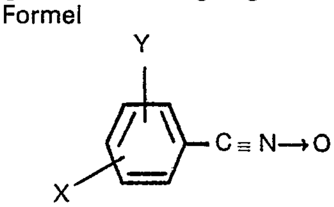

mit 3-Methylenphthalid in einem inerten Lösungsmittel umfaßt.

30. Verfahren nach Anspruch 29, worin das Nitriloxid in situ durch Zugabe eines Chloroxims der Formel

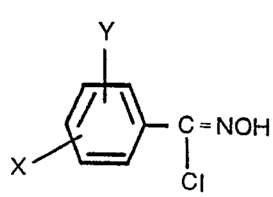

und einer Base zu der 3-Methylenphthalid-Lösung herstellt wird.

31. Verfahren nach Anspruch 29, worin die Base ein tertiäres Amin ist.

32. Verfahren nach Anspruch 31, worin das Amin Triethylamin ist.

33. Verfahren zur Herstellung von 3-Aryl-isoxazol-5-yl-benzoesäure und deren Ester der Formel

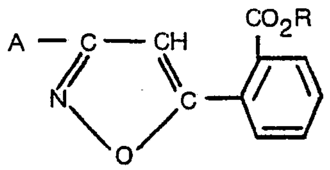

worin R ein Wasserstoffatom oder den Rest $R'CH_2$— bedeutet und R' ein Wasserstoffatom oder einen Alkylrest mit bis zu 4 Kohlenstoffatomen darstellt, A Pentafluorphenyl ist oder die Gruppe

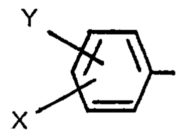

bedeutet, worin X und Y unabhängig voneinander ein Wasserstoff- oder Halogenatom oder einen $C_{1-5}$-Alkyl-, $C_{1-5}$-Alkoxy-, Halogen-$C_{1-5}$-alkyl-, Phenoxy- oder Phenylrest darstellen, wobei in Gegenwart einer katalytischen Menge einer starken Säure eine Spiro-Verbindung der Formel

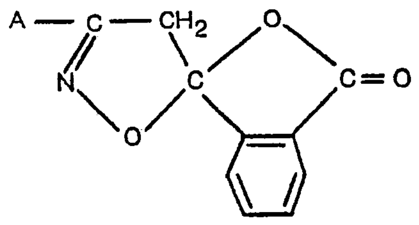

worin A, X und Y die vorstehend genannte Bedeutung haben, mit ROH bei einer Temperatur im Bereich von der Raumtemperatur bis zur Rückflußtemperatur der ROH-Verbindung umgesetzt wird.

34. Verfahren nach Anspruch 33, worin die Temperatur die Rückflußtemperatur der ROH-Verbindung ist.

35. Verfahren nach Anspruch 33, worin R die Gruppe $R'CH_2$— darstellt.

36. Verfahren nach Anspruch 33, worin Y die Trifluormethylgruppe ist.

37. Verfahren nach Anspruch 33, worin die starke Säure Schwefelsäure ist.